# EUROPEAN PATENT SPECIFICATION

(11) **EP 0 670 711 B1**
(45) Date of publication and mention of the grant of the patent: **17.02.1999**
(21) Application number: 94900238.0
(22) Date of filing: 19.11.1993
(51) Int. Cl.: A61K 7/16, A61K 38/00

(54) **ANTIFUNGAL MOUTHCARE COMPOSITIONS**
ANTIFUNGALE MUNDPFLEGE ZUSAMMENSETZUNGEN
COMPOSITIONS D'HYGIENE BUCCALE ANTIFONGIQUES

(30) Priority: 24.11.1992 GB 9224598
(43) Date of publication of application: 13.09.1995
(73) Proprietor: AMBI Inc., Tarrytown, NY 10591 (US)
(72) Inventor: FORWARD, Geoffrey Charles, SmithKline Beecham, Weybridge, Surrey KT13 0DE (GB); BARTLETT, Michael Edwin, SmithKline Beecham, Weybridge, Surrey KT13 0DE (GB); MCCONVILLE, Peter Scott, SmithKline Beecham, Weybridge, Surrey KT13 0DE (GB)
(74) Representative: Lucas, Brian Ronald
(86) International application number: GB9302387
(87) International publication number: WO9412150

(56) References cited:
- EP-A- 0 140 498
- EP-A- 0 545 911
- WO-A-90/09739
- CA-A- 2 055 984
- CA-A- 2 058 455
- DE-A- 2 755 052
- GB-A- 2 160 771

## Description

This invention relates to the manufacture of oral hygiene compositions and in particular to mouthwashes and dentifrices having improved activity against fungi. Pathogenic fungi such as *Candida* may also be present in the oral cavity and give rise to disease states such as thrush requiring therapy.

Agents which in the past have been suggested for use as oral antibacterial agents include cationic species such as chlorhexidine, alexidine, hexetidine and cetyl pyridinium chloride as well as non-cationic species such as triclosan. Some of these antibacterial agents are also effective as antifungal agents.

More recently, it has been suggested that the polypeptide antibiotic nisin (Merck Index, 11th edn., entry 6481) may be of use in oral hygiene. Nisin is a lanthocin, comprising the atypical amino acid lanthionine, produced naturally by various stains of the bacterium *Streptococcus lactis*. It is also a naturally occurring preservative found in low concentration in milk and cheese. Nisin has recently been recognised by the FDA as a direct food ingredient. A summary of the properties of nisin is to be found in Advances in Applied Microbiology 27 (1981), 85-123. A purified form of nisin has recently been made available by Applied Microbiology Inc., under the trade name Ambicin N.

Oral care applications of nisin are disclosed in WO 93/11738 and WO 89/12399. The former discloses suitable dentifrice formulations whilst the latter discloses broad spectrum disinfectant compositions which may be in the form of oral rinses in which nisin is combined with a non-bactericidal agent such as a surfactant or a chelating agent, to extend the spectrum of activity of the composition.

In many instances, a single anti-microbial agent does not have a sufficiently broad spectrum of activity to deal adequately with a wide range of pathogenic microorganisms which may be found in the oral cavity. Combining different antimicrobial agents is not always successful as the presence of one may antagonise the activity of the other.

WO 90/09739 discloses compositions comprising nisin and lysostaphin as a broad range bactericide, primarily for food-related uses, but the possibility of use as an oral rinse is also mentioned.

We have now surprisingly found that nisin may be effectively combined with certain other antimicrobial agents, in formulating oral care compositions for treating or prophylaxis of fungal infections, without compromising the activity of nisin or the antimicrobial agent.

Accordingly, the present invention provides the use of nisin, an antimicrobial agent selected from chlorhexidine or a salt thereof, cetyl pyridinium chloride or triclosan and an orally acceptable excipient or carrier in the manufacture of an oral care composition for treating or the prophylaxis of oral fungal infection.

The compositions to which the present invention relates have a broader range of antimicrobial activity. For instance, nisin has limited activity against *Candida* species whereas chlorhexidine, cetyl pyridinium chloride or triclosan has anti-*Candida* activity which is maintained in the presence of nisin.

Suitably, in the context of the present invention, nisin is used in a purified form, for instance the product sold under the trade name Ambicin N by Applied Microbiology Inc., 170 53rd Street, Brooklyn, New York, NY 11232, USA.

Suitably, the oral care composition comprises from 0.001 to 5.0%, preferably from 0.005 to 2.0%, advantageously from 0.02 to 1.0 % of nisin, by weight of the composition. In an alternative manner, the level of nisin needed is one which reaches a sufficent level in the oral cavity to inhibit the desired microrganisms. An effective level of nisin, to inhibit the desired organisms, is about 0.99 ppm.

Typically, the chlorhexidine or cetyl pyridinium chloride will be present in the range 0.005 to 10%, preferably 0.005 to 5%, more preferably 0.005 to 2.5% by weight of the oral hygiene composition.

Typically, triclosan, a water insoluble noncationic antimicrobial agent, will be present in the range 0.005 to 2%, preferably 0.005 to 1%, and more preferably from 0.005 to 0.3% by weight of the composition.

Antimicrobial agents such as chlorhexidine, cetyl pyridinium chloride and triclosan are active against both bacteria and fungi. In many instances it is found that the concentration of the antimicrobial agent required for antifungal activity is less than that required for antibacterial activity. For instance, at a concentration of about 8.15ppm, cetyl pyridinum chloride kills all the organisms shown in the Example 25 Table 1. In comparison, a level of only about 1.39ppm cetyl pyridinum chloride is needed to inhibit *Candida* organisms. Similarly for chlorhexidine and triclosan, the levels needed to inhibit all the desired oral microrganisms are about 8.46ppm and 7.65ppm respectively, whilst the levels for *Candida* inhibition are 1.71ppm and about 1ppm, respectively. Thus, lower levels of such antimicrobial agent may be used in the present invention if it intended that such antimicrobial agent is being included principally to provide anti-Candida activity, to supplement the gap in the spectrum of activity of nisin.

Oral hygiene compositions of the present invention may be presented in any of the formulations conventionally used in the art; for instance, as a mouthwash, dentifrice, including toothpaste and toothpowder liquid toothpaste, gel, tablet, lozenge or chewing gum. The components for the orally acceptable carrier or excipient will be selelected according to principles well known to those skilled in the art for the preparation of such formulation types. Such components include a surfactant, thickening agent, humectant and abrasive, as appropriate, as well as other optional extras normally included in an oral care composition. Such components should be compatible with nisin and the antimicrobial agent. Thus, it has previously been found (WO 93/11738, SmithKline Beecham) that polypeptide antibiotics such as nisin are incompatible with anionic surfactants such as sodium lauryl sulphate and sodium N-methyl-N-cocyl laurate which are conventionally used in oral care compositions. Such anionic surfactants should preferably be avoided, in favour of nonionic, cationic or amphoteric surfactants or a mixture thereof.

Similarly, it is recognised by those skilled in the art that chlorhexidine and, to a lesser extent, cetyl pyridinium chloride present formulation problems because of their incompatibility with anionic species normally used in the formulation of oral care compositions, in particular anionic surfactants and, at least for chlorhexidine, anionic thickening agents. These should preferably be avoided. In addition, for dentifrices, care needs to be taken in selecting suitable abrasives, as herein after described.

The term "compatible", when used herein with reference to the selection of an additional formulation ingredient, is used to indicate that the activity of the antifungal agent, is not substantially compromised by the presence of the ingredient. Suitably that activity in the presence of the ingredient should not be less than 40%, preferably less than 50%, advantageously less than 60% of that observed in the absence of the ingredient. This may be readily checked by bioassay, for instance a conventional zone diffusion assay against an organism sensitive to that agent, for instance, *Micrococcus luteus* NCTC 8166 (for triclosan).

Suitable nonionic surfactants include, for example, polyethoxylated sorbitol esters, in particular polyethoxylated sorbitol monoesters, for instance, PEG(40) sorbitan diisostearate, and the products marketed under the Registered Trade Mark 'Tween' by ICI; polycondensates of ethylene oxide and propylene oxide (poloxamers), for instance the products marketed under the Registered Trade Mark 'Pluronic; by BASF-Wyandotte; condensates of propylene glycol; polyethoxylated hydrogenated castor oil, for instance, cremophors; and sorbitan fatty esters.

Suitable amphoteric surfactants include, for example, long chain imidazoline derivatives such as the product marketed under the trade name 'Miranol C2M' by Miranol; long chain alkyl betaines, such as the product marketed under the tradename 'Empigen BB' by Albright + Wilson, and long chain alkyl amidoalkyl betaines, such as cocamidopropylbetaine, and mixtures thereof.

Suitable cationic surfactants include the D,L-2-pyrrolidone-5-carboxylic acid salt of ethyl-N-cocoyl-L-arginate, marketed under the trade name CAE by Ajinomoto Co. Inc., and cocamidopropyl PG dimonium chloride phosphate and lauramidopropyl PG dimonium chloride phosphate, available under the trade names Monaquat PTC and Monaquat PTL, respectively, from Mona Corporation.

Advantageously, the surfactant is present in the range 0.005 to 20%, preferably 0.1 to 10%, more preferably 0.1 to 5% by weight of the dentifrice.

Suitable thickening agents include, for instance, nonionic thickening agents such as, for example, (C₁-₆)alkylcellulose ethers, for instance methylcellulose; hydroxy(C₁-₆)alkylcellulose ethers, for instance hydroxyethylcellulose and hydroxypropylcellulose; (C₂-₆)alkylene oxide modified (C₁-₆)alkylcellulose ethers, for instance hydroxypropyl methylcellulose; and mixtures thereof. Other thickening agents such as natural and synthetic gums or gum like material such as Irish Moss, gum tragacanth, sodium carboxymethylcellulose, polyvinylpyrrolidone, starch and thickening silicas are suitable for use in many of the compositions of the present invention, although their use should be avoided in compositions comprising chlorhexidine. Suitably the thickening agent has decreased numbers of anionic groups, such as a carboxy group, although carboxymethyl cellulose may be used. Preferably, the thickening agent is a methylcellulose derivative such as hydroxyethyl cellulose, or hydroxypropyl methylcellulose.

Advantageously the thickening agent is present in the range 0.01 to 30%, preferably 0.1 to 15%, more preferbly 1 to 5%, by weight of the composition.

Suitable humectants for use in compositions of the invention include for instance, glycerine, sorbitol, propylene glycol or polyethylene glycol, or mixtures thereof; which humectant may be present in the range from 5 to 70%, preferably 5 to 30%, more preferably 10 to 30% by weight of the dentifrice. Suitably, when the nonionic thickening agent is hydroxypropyl methylcellulose, the humectant is present in up to 30% by weight of the dentifrice.

Suitable abrasives for use in dentifrice compositions of the present invention include calcium carbonate, calcium phosphates, calcium pyrophosphate, insoluble sodium metaphosphate, sodium aluminosilicate, alumina, hydrated alumina, zinc orthophosphate, plastic particles, and silica, of which silica is the preferred abrasive.

Suitable silicas include natural amorphous silicas, such as, for instance, diatomaceous earth, and synthetic amorphous silicas, such as precipitated silicas and silica gels, including silica xerogels. Suitable silica xerogels are described in US 3,538,230. Suitable grades of precipitated silicas have BET surface areas in the range 20 to 300, preferably 20 to 100 m²/g and median agglomerate sizes in the range 2 to 50, preferably 5 to 30µ.

Suitable precipitated silicas and silica xerogels are those marketed under the trade names Sident and Syloblanc, by Degussa and W R Grace Corporation Davison Chemical Division, respectively.

Advantageously, the silica is a "low anion" silica. As used herein, the term "low-anion" silicas refers to those in which anionic impurities such as sodium sulphate and sodium silicate which normally arise during the course of the manufacturing process are kept to a minium, through careful control of the manufacturing process. "Low anion" silicas suitably have less than 1%, preferably less than 0.5% advantageously less than 0.25% by weight of anionic impurities.

Suitable such "low anion" silicas are described in EP 0 368 130 (Proctor & Gamble), EP 0 315 503 and EP 0 396 459 (Rhone-Poulenc) and WO 90/05113 (J.M. Huber Corp). Alternatively, grades of commercially available silica with ionic impurities may be rendered suitable by washing thereof with deionised water. Conductivity measurements on the water after washing may be used to monitor the efficacy of such washing. Suitably the conductivity of the water after washing is reduced to less than 200µSiemens/cm. Suitable "low anion" silicas include the grade RP93 available from Rhone-Poulenc.

Suitably, when the additional antimicrobial agent is chlorhexidine, and the abrasive is a silica derivative, the preferred silica is of the "low anion" type.

Suitably, compositions will have from 5 to 80%, preferably from 10 to 60% by weight of the abrasive.

Suitable dentifrice formulations for compositions comprising chlorhexidine which may be adapted for compositions comprising nisin and chlorhexidine are described in EP 0 364 245-A and EP 0 422 803-A (Beecham Group plc) and EP 0 368 130 (Proctor & Gamble). Suitable dentifrice formulations for compositions comprising cetyl pyridinium chloride which may be adapted for compositions comprising nisin and cetyl pyridinium chloride are described in US 5 176 901 (SmithKline Beecham Corporation).

In a preferred aspect, dentifrice compositions according to the present invention comprise nisin, preferably in the form Ambicin N; an antimicrobial agent selected from cetyl pyridinium chloride, a chlorhexidine salt or triclosan, preferably a chlorhexidine salt or triclosan; a nonionic surfactant such as, for instance, a polycondensate of ethylene oxide and propylene oxide; a nonionic thickening agent such as, for instance, hydroxypropyl methylcellulose; a humectant such as, for instance, glycerin; and an abrasive such as for instance a "low-anion" silica or a calcium carbonate, optionally in combination with dicalcium *ortho*monophosphate and including an alkaline earth metal metal salt such as calcium chloride.

In a further preferred aspect, a dentifrice composition manufactured according to the present invention comprises nisin, an antimicrobial agent selected from cetyl pyridinium chloride or triclosan, a nonionic surfactant such as, for instance, a polycondensate of ethylene oxide and propylene oxide, a thickening agent which is sodium carboxymethyl cellulose optionally admixed with a thickening silica, a humectant such as sorbitol optionally admixed with glycerin, and an abrasive which is a "low anion" silica.

Suitable mouthwash formulations will have an aqueous base comprising water or aqueous ethanol, and optionally a further liquid such as glycerin or propylene glycol. A surfactant may also be included, to improve the sensory properties of the composition. Mouthwash compositions may be provided in a "ready to use" form; as a concentrated solution, for dilution by the user immediately prior to use; or in solid form, such as a tablet or in a sachet, for dissolution by the user immediately prior to use. Tablets may suitably be prepared using xylitol and/or sorbitol as the major ingredient. The sachets and tablets may be formulated to provide, on dissolution, a still mouthwash, or, by the incorporation of a suitable effervescent couple, for instance sodium carbonate/bicarbonatre and citric acid, an effervescent mouthwash.

Compositions manufactured according to the present invention may usefully comprise a fluoride ion source, to provide an anti-caries activity. A fluoride ion source is found to be compatible with nisin. The appropriate fluoride source for the combination of nisin and antimicrobial agent will depend upon the particular antimicrobial agent chosen. The compatabilities, and incompatabilities are well known and documented in the art for each of the suitable antimicrobial agent. Where applicable, therefore, suitable fluoride ion sources include metal fluoride salts, for instance alkali metal fluoride salts such as sodium fluoride, amine fluoride salts, alkali metal monofluorophosphate salts such as sodium monofluorophosphate and amine monofluorophosphate salts. Suitably the fluoride ion source would, if present, be included to provide from 50 to 3500 ppm, preferably 100 to 2500 ppm of fluoride ions.

In addition to a humectant, compositions manufactured according to the present invention may also contain further liquid such as, for instance, water, preferably deionised water.

Compositions manufactured according to the present invention may usefully comprise an orally acceptable chelating agent such as EDTA or citric acid or an alkali metal salt thereof, for instance disodium hydrogen citrate, in accordance with the disclosure of WO 89/12399 (Public Health Research Institute of the City of New York). Suitably, nisin is present in a concentration of from 0.1 to 300µg/ml and the chelating agent present in a concentration of from 0.1 to 20mM.

Nisin comprises the atypical amino acid lanthionine which may be conveniently regarded as two alanine units bonded to a common sulphur atom, to form a thioetther link. This linkage may be vulnerable to proteolysis, leading to deactivation. This is thought to be caused by free radicals which may be generated by certain components of the oral hygiene composition, in particular impurities which may be present in certain components. We have found that some of the grades of nonionic surfactants like the Tweens and some grades of some humectants may cause such a problem. The use of purified grades of formulation ingredients is therefore preferred. In addition, or as an alternative, a competitive substrate, to act as a free radical scavanger, may be usefully included in the composition, for instance, methionine.

The orally acceptable vehicle or carrier may also comprise further optional ingredients such as flavouring agents, sweetening agents, for example sodium saccharin, dyes, whitening agents, for example titanium dioxide, preservatives, antisensitivity agents, such as stronium and potassium salts, and anticalculus agents, such as tetraalkali- and dialkali-imetal pyrophosphate salts. It will be appreciated that in each instance, an optional ingredient, if included, will be compatible with nisin and the antimicrobial agent. It is further appreciated that the combination of additional ingredients such as stronium will be in a manner compatible with other ingredients such as fluoride ion source.

Compositions manufactured according to the invention will have a pH which is orally acceptable and within which the antibacterial activity of nisin is not substantially compromised. Suitably, the pH is in the range 4 to 9.5, preferably in the range 4 to 6.5, more preferably between 4 and 5.5 and most preferably 5 to 5.5.

Compositions manufactured according to the invention may be prepared by conventional processes comprising admixing the ingredients together in the appropriate relative amounts in any order that is convenient and finally, and if necessary, adjusting the pH to the desired value.

Compositions of the present invention are intended for use in the prophyllaxis and/or treatment of the diseases within the oral cavity. In particular, compositions of the present invention are effective against oral plaque bacterial and as such will be of use in antiplaque therapy. Accordingly, in a further aspect, the present invention also provides a method of reducing or preventing the formulation of dental plaque, which method comprises applying an antiplaque effective amount of a composition according to the present invention to a patient in need thereof. Compositions of the present invention are also useful in the prophyllaxis or treatment of periodontal disease, including gingivitis. Accordingly, the present invention also provides for a method of treating or prophyllaxis of periodontal disease. Certain compositions of the present invention as hereinbefore defined are also of use in treating oral fungal infections. Accordingly, the present invention also provides for a method of treating or prophyllaxis of oral fungal infection.

The invention will now be illustrated by reference to the following examples.

### Example 1 - Toothpaste

| | |
|---|---|
| Ambicin N | 0.50% |
| Triclosan | 0.2 |
| Glycerin | 22.00 |
| Hydroxypropyl methylcellulose | 3.40 |
| Titanium dioxide | 1.00 |
| Sodium saccharin | 0.25 |
| Poloxamer (Pluronic F108) | 2.00 |
| Flavour | 1.00 |
| Silica (RP93) | 16.00 |
| Deionised water | qs |

Further examples can be prepared using 0.1 or 0.3% triclosan.

### Example 2 - Toothpaste

| | |
|---|---|
| Ambicin N | 0.50% |
| Chlorhexidine digluconate | 0.5 |
| Glycerin | 22.00 |
| Hydroxypropyl methylcellulose | 3.40 |
| Titanium dioxide | 1.00 |
| Sodium saccharin | 0.10 |
| Poloxamer (Pluronic F108) | 2.00 |
| Flavour | 1.00 |
| Talin | 0.02 |
| Silica (RP93) | 16.00 |
| Deionised water | qs |

Further examples can be prepared using 0.05 or 1.0% chlorhexidine digluconate.

### Example 3 - Toothpaste

| | |
|---|---|
| Ambicin N | 0.50% |
| Cetyl pyridinium chloride | 0.5 |
| Glycerin | 22.00 |
| Hydroxypropyl methylcellulose | 3.40 |
| Titanium dioxide | 1.00 |
| Sodium saccharin | 0.10 |
| Poloxamer (Pluronic F108) | 2.00 |
| Flavour | 1.00 |
| Talin | 0.02 |
| Silica (RP93) | 16.00 |
| Deionised water | qs |

Further examples can be prepared using 0.05 % and 1.0 % cetyl pyridinium chloride.

### Example 4 - Toothpaste

| | |
|---|---|
| Ambicin N | 0.05% |
| Triclosan | 0.3 |
| Sorbitol (70% soln) | 20.00 |
| Glycerin | 15.00 |
| Sodium carboxymethyl cellulose | 1.20 |
| Sodium fluoride | 0.23 |
| Silica (RP 93) | 16.00 |
| Thickening silica (Sident 22) | 5.00 |
| Sodium saccharin | 0.30 |
| Poloxamer (Pluronic F108) | 2.00 |
| Deionised water | qs |

Further examples can be prepared using 0.1 or 0.2% triclosan.

### Example 5 - Toothpaste

| | |
|---|---|
| Ambicin N | 0.05% |
| Cetyl pyridinium chloride | 1.0 |
| Sorbitol (70% soln) | 20.00 |
| Glycerin | 15.00 |
| Sodium carboxymethyl cellulose | 1.20 |
| Sodium fluoride | 0.23 |
| Silica (RP 93) | 16.00 |
| Thickening silica (Sident 22) | 5.00 |
| Sodium saccharin | 0.30 |
| Poloxamer (Pluronic F108) | 2.00 |
| Deionised water | qs |

Further examples can be prepared using 0.05 or 0.5% cetyl pyridinium chloride.

### Example 6 - Toothpaste

| | |
|---|---|
| Ambicin N | 0.05% |
| Triclosan | 0.2 |
| Glycerin | 22.00 |
| Methocel K15 Premium | 0.20 |
| Methocel K100 Premium | 3.20 |
| Titanium dioxide | 1.00 |
| Sodium saccharin | 0.33 |
| Poloxamer (20% Pluronic F108 soln) | 10.00 |
| Sodium Fluoride | 0.221 |
| Flavour | 1.00 |
| Silica (RP 93) | 16.00 |
| Deionised water | qs |

A further example can be prepared by replacing Methocel K15 and Methocel K100 by sodium carboxymethyl cellulose (1.20%). Additionally, the level of triclosan can be varied using 0.1% or 0.3%.

### Example 7 - Toothpaste

| | |
|---|---|
| Ambicin N | 0.05% |
| Chlorhexidine digluconate | 1.0 |
| Glycerin | 22.00 |
| Methocel K15 Premium | 0.20 |
| Methocel K100 Premium | 3.20 |
| Titanium dioxide | 1.00 |
| Sodium saccharin (30% soln) | 0.33 |
| Poloxamer (20% Pluronic F108 soln) | 10.00 |
| Sodium Fluoride | 0.22 |
| Flavour | 1.00 |
| Talin (5% soln) | 0.40 |
| Silica (RP 93) | 16.00 |
| Deionised water | qs |

A further example can be prepered by replacing the Methocel K15 and Methocel K100 with sodium carboxymethyl cellulose (1.20%). Additionally, the level of chlorhexidine digluconate can be varied using 1.0 % or 0.05 %.

### Example 8 - Toothpaste

| | |
|---|---|
| Ambicin N | 0.05% |
| Cetyl pyridinium chloride | 1.0 |
| Glycerin | 22.00 |
| Methocel K15 Premium | 0.20 |
| Methocel K100 Premium | 3.20 |
| Titanium dioxide | 1.00 |
| Sodium saccharin (30% soln) | 0.33 |
| Poloxamer (20% Pluronic F108 soln) | 10.00 |
| Sodium Fluoride | 0.221 |
| Flavour | 1.00 |
| Talin (5% soln) | 0.40 |
| Silica (RP 93) | 16.00 |
| Deionised water | qs |

A further example can be prepared by replacing the Methocel K15 and Methocel K100 with sodium carboxymethyl cellulose (1.20%). Additionally, the level of cetyl pyridinium chloride can be varied using 0.05 % or 0.5 %.

### Example 9 - Mouthwash

| | |
|---|---|
| Ambicin N | 0.030% |
| Triclosan | 0.020 |
| Glycerin | 5.000 |
| Flavour | 0.075 |
| Ethanol (96%) | 15.00 |
| Soluble saccharin | 0.010 |
| Sodium Fluoride | 0.023 |
| Colouring | 0.100 |
| Propylene Glycol | 15.00 |
| Deionised water | qs |

Further examples can be prepared using triclosan at 0.01% or 0.03%.

### Example 10 - Mouthwash

| | |
|---|---|
| Ambicin N | 0.030 % |
| Triclosan | 0.03 |
| Glycerin | 30.000 |
| Flavour | 0.075 |
| Ethanol (96%) | 15.00 |
| Cremophor RH60 | 0.60 |
| Soluble saccharin | 0.005 |
| Sodium Fluoride | 0.023 |
| Colouring | 0.10 |
| Deionised water | qs |

In an alternative formulation, Cremophor RH60 may be replaced by propylene glycol. Further, the level of triclosan may also be varied using 0.005, 0.01% or 0.02%.

### Example 11 - Mouthwash

| | |
|---|---|
| Ambicin N | 0.030% |
| Chlorhexidine digluconate | 0.01 |
| Glycerin | 5.000 |
| Flavour | 0.075 |
| Ethanol (96 %) | 5.00 |
| Soluble saccharin | 0.010 |
| Sodium Fluoride | 0.023 |
| Colouring | 0.100 |
| Cremophor RH60 | 0.1 |
| Deionised water | qs |

A further example may be prepared using Cremophor RH60 at 0.2%. In addition, the level of chlorhexidine digluconate may be varied, using 0.05%, 0.1% or 0.2 %.

### Example 12 - Mouthwash

| | |
|---|---|
| Ambicin N | 0.030% |
| Cetyl pyridinium chloride | 0.01% |
| Glycerin | 5.000 |
| Flavour | 0.075 |
| Ethanol (96%) | 5.00 |
| Soluble saccharin | 0.0001 |
| Sodium Fluoride | 0.023 |
| Colouring | 0.001 |
| Cremophor RH60 | 0.1 |
| EDTA | 0.004 |
| Deionised water | qs |

A further examples may be prepared using Cremophor RH60 at 0.2%. In addition, Cremophor RH60 may be omitted or replaced by a nonionic surfactant such as Tween 20 or TritonX100 (0.2%). Other examples can be prepared using 0.05, 0.1 or 0.2 % cetyl pyridinium chloride.

### Example 13 -Mouthwash

| | |
|---|---|
| Ambicin N | 0.030% |
| Triclosan | 0.005 |
| Disodium hydrogen citrate | 0.263 |
| Ethanol (96%) | 15.00 |
| Propylene glycol | 15.00 |
| Glycerin | 30.00 |
| Deionised water | qs |

Further examples may be prepared in which the level of triclosan is 0.01%, 0.02%, or 0.03%.

### Example 14 - Mouthwash

| | |
|---|---|
| Ambicin N | 0.030% |
| Chlorhexidine digluconate | 0.005 |
| Disodium hydrogen citrate | 0.263 |
| Deionised water | qs |

Further examples may be prepared using chlorhexidine digluconate at 0.05%, 0.1% or 0.2% levels.

### Example 15 - Mouthwash

| | |
|---|---|
| Ambicin N | 0.030% |
| Cetyl pyridinium chloride | 0.01 |
| Disodium hydrogen citrate | 0.263 |
| Deionised water | qs |

Further examples may be prepared using 0.05, 0.1 or 0.2% of cetyl pyridinium chloride.

### Example 16 - Mouthwash (concentrated formulation)

| | |
|---|---|
| Ambicin N | 1.0% |
| Cetyl pyridinium chloride | 0.75 |
| Propylene glycol | 73.00 |
| Ethanol (96%) | 15.00 |
| Flavour | 2.91 |
| Sodium Saccharin | 0.11 |
| Tween 20 | 0.5 |
| Deionised water | 6.48 |

The concentrated mouthwash is used by diluting a few drops (about 1ml) into a half-filled glass of water (approx. 100ml) which is gargled by the user.

### Example 17 - Mouthwash (concentrated formulation)

Using the formulation of example 16 but with cetyl pyridinium chloride at between 0.075 to 1.5%.

### Example 18 - Mouthwash (concentrated formulation)

Using the formulation of example 16 but replacing phenyl salicylate with chlorhexidine digluconate at between 0.075 to 1.5%, for instance 0.75%.

### Example 19 - Mouthwash tablet

| | |
|---|---|
| Ambicin N | 0.9% |
| Triclosan | 0.9 |
| Sodium Fluoride | 1.5 |
| Magnesium Stearate | 1.0 |
| Flavour (spray dried) | 1.5 |
| Colouring | 0.05 |
| Citric Acid (anhydrous) | 15 |
| Sodium Bicarbonate | 11 |
| Sodium Carbonate | 10 |
| Xylitol | qs |

Further examples may be prepared using replacing xylitol with sorbitol or a mixture of xylitol and sorbitol. The ingredients are admixed and compressed into 500mg tablets. The citric acid, sodium bicarbonate and sodium carbonate are preferably admixed with the xylitol or sorbitol first. Further examples may be prepared using 0.1% triclosan. In use, the tablet is disolved in about 100ml of water, to form an effervescent mouthwash solution which is then gargled by the user.

### Example 20 - Mouthwash tablet

Using the formulation of example 19 but replacing triclosan with chlorhexidine acetate at 0.9 or 0.075 %.

### Example 21 - Mouthwash tablet

Using the formulation of example 19 but replacing triclosan by cetyl pyridinium chloride at 1.5 or 0.075 %.

### Example 22 - Sachet - Effervescent Mouthwash

| | |
|---|---|
| Ambicin N | 0.225% |
| Triclosan | 0.225 |
| Sodium Fluoride | .375 |
| Menthol | 0.5 |
| Citric Acid (anhydrous) | 15.0 |
| Sodium Bicarbonate | 11.0 |
| Sodium Carbonate | 10.0 |
| Xylitol | qs to 100 |

Xylitol may be replaced by sorbitol or a sorbitol/xylitol mixture. The ingredients are admixed and placed into 2 g dosage packets. The sodium bicarbonate and sodium carbonate are preferably admixed separately with xylitol or sorbitol first. The level of the effervescent couple may be increased to citric acid (22.5%), sodium bicarbonate (16.5%), and sodium carbonate (15%). Further examples may be prepared in which the level of triclosan is 0.0225%.

### Example 23 - Sachet - Effervescent Mouthwash

Using the formulations of example 22 but replacing triclosan by cetyl pyridinium chloride at from 0.05 to 0.375 %.

### Example 24 - Sachet - Effervescent Mouthwash

Using the formulations of example 22 but replacing triclosan with chlorhexidine acetate at from 0.025 to 0.225%.

### Example 25 - Antibactetial and antifungal spectrum of the active ingredients

The antibacterial spectrum of activity of Ambicin N (Amb) was determined by testing the compound against a range of orally important Gram negative and Gram positive bacteria in a conventional nominal inhibitory concentration (NIC) assay and compared with that of cetyl pyridinium chloride (CPC), chlorhexidine (CHX) and triclosan. The results are reported in Table 1. In most instances, Ambicin N has superior activity but as can also be noted Ambicin N lacks effective inhibitory activity against *candida* species. In comparison, CPC, CHX and TCN are all effective against *Candida*.

**Table 1**

| **NIC data** | | | | |
|---|---|---|---|---|
| Organism | CPC (ppm) | CHX (ppm) | TCN (ppm) | Amb (ppm) |
| Strep. agalactiæ | 0.78 | 0.41 | 4.96 | 0.22 |
| Strep. sanguis | 2.12 | 0.86 | 0.88 | 0.23 |
| Strep.mutans | 3.10 | 3.94 | 1.11 | 0.43 |
| Strep. milleri | 4.58 | 1.58 | 1.08 | 0.14 |
| Strep. mitis | 2.23 | 1.50 | 3.94 | 0.33 |
| Strep mitior | 1.22 | 4.55 | 4.23 | 0.05 |
| Strep. salivarius | 2.36 | 1.44 | 4.05 | 0.18 |
| Strep.pyogenes | 0.78 | 0.16 | 4.35 | 0.009 |
| Staph.aureus | 0.66 | 0.54 | 0.09 | 0.13 |
| G.vaginalis | 0.3 | 1.3 | 0.41 | 0.22 |
| Lacto odontolyticus | 0.83 | 1.88 | 7.49 | 0.02 |
| Act. odontolyticus | 1.23 | 3.37 | 3.02 | 0.12 |
| Act. israelii | 8.15 | 4.11 | 3.19 | 0.53 |
| Act. naeslundii | 4.61 | 2.01 | 1.98 | >12.8 |
| Act. actinomycetem | 1.02 | 0.53 | 1.69 | 0.02 |
| Fuso. nucleatum | 0.82 | 0.64 | 1.6 | 0.048 |
| Bact. intermedius | 4.68 | 2.49 | 3.72 | 0.91 |
| Peptostrepto. micros | 4.96 | 8.46 | 6.76 | >1.28 |
| Porph. gingivalis | 0.94 | 2.36 | 4.00 | >1.28 |
| Bact. ureolyticus | 0.56 | 0.69 | 7.65 | 0.035 |
| Candida albicans | 1.39 | 1.71 | 1.00 | >128 |
| Candida kefyr | 0.61 | 0.42 | 0.46 | >128 |
| Candida tropicalis | 0.48 | 0.98 | 0.35 | >128 |

### Example 26 - Antibacterial and antifungal spectrum of the active ingredients

The antibacterial activity of a solution comprising Ambicin N (Amb) (500ppm) in the presence or absence of one of cetyl pyridinium chloride (CPC), chlorhexidine (CHX) or triclosan (TCN) (500ppm) was assayed in a conventional zone difusion assay against the important oral microorganisms *S sanguis*, *S mutans*, *A actionmyces*, *W* *recta*, *F nucleatum*, *C albicans*, *C kefyr* and *C tropicalis*. The assay was repeated for each of cetyl pyridinium chloride, chlorhexidine or triclosan individually. The results are presented in tables 2 and 3. Although there is no evidence to suggest any degree of synergy between Ambicin N and any of cetyl pyridinium chloride, chlorhexidine or triclosan, the data also shows that there is no reduction in the activity of either agent in the presence of the other; ie, there is mutual compatibility.

**Table 2**

| **Zone Diffusion Assay - mean zones obtained against various oral bacteria (mm)** | | | | | |
|---|---|---|---|---|---|
| | **S sanguis** NCTC 10904 | **S mutans** NCTC 11061 | **A actinomyces** NCTC 9709 | **W recta** NCTC 11489 | **F nucleatum** NCTC 10562 |
| Ambicin N | 15.38 | 18.36 | 23.04 | 28.19 | 29.38 |
| TCN | 28.97 | 32.82 | 35.92 | 37.20 | 33.98 |
| CPC | 14.44 | 17.05 | 19.41 | 17.15 | 18.99 |
| CHX | 22.76 | 29.68 | 35.68 | 32.35 | 36.97 |
| Amb/TCN | 26.73 | 31.47 | 31.28 | 33.08 | 26.11 |
| Amb/CPC | 21.92 | 29.42 | 35.25 | 31.52 | 37.33 |
| Amb/CHX | 14.48 | 17.05 | 19.57 | 20.48 | 19.25 |

**Table 3**

| **Zone Diffusion Assay - mean zones obtained against various** ***Candida*** **sp (mm)** | | | |
|---|---|---|---|
| | **C kefyr** NCTC 3106 | **C tropicalis** NCTC 3114 | **C albicans** NCTC 3089 |
| Ambicin N (Amb) | 0 | 0 | 0 |
| TCN | 21.99 | 13.66 | 16.99 |
| CPC | 10.43 | 10.39 | 9.79 |
| CHX | 17.03 | 17.36 | 14.93 |
| Amb/TCN | 18.66 | 11.77 | 16.74 |
| Amb/CPC | 16.21 | 17.45 | 14.58 |
| Amb/CHX | 10.66 | 9.98 | 9.74 |

## Claims

1. The use of nisin, an antimicrobial agent selected from chlorhexidine or a salt thereof, cetyl pyridinium chloride or triclosan and an orally acceptable excipient or carrier in the manufacture of an oral care composition for treating or the prophylaxis of oral fungal infection.

2. The use as claimed in Claim 1 in which nisin is used in a purified form.

3. The use as claimed in Claim 1 or 2 in which the orally acceptable carrier or excipient comprises a surfactant which is a nonionic, cationic or amphoteric surfactant or a mixture thereof.

4. The use as claimed in any one of Claims 1 to 3 in which the orally acceptable carrier or excipient comprises a nonionic thickening agent or a natural or synthetic gum or gum-like material.

5. The use as claimed in any one of Claims 1 to 4 in which the composition is a dentifrice and in which the abrasive is a silica abrasive.

6. The use as claimed in any one of Claims 1 to 4 in which the composition is a dentifrice which comprises nisin; an antimicrobial agent selected from cetyl pyridinium chloride, a chlorhexidine salt or triclosan; a nonionic surfactant; a nonionic thickening agent; a humectant; and an abrasive which is a "low-anion" silica or calcium carbonate optionally in combination with dicalcium *ortho*monophosphate and including an alkaline earth metal metal salt such as calcium chloride.

7. The use as claimed in any one of Claims 1 to 4 in which the composition is a dentifrice which comprises nisin; an antimicrobial agent selected from cetyl pyridinium chloride or triclosan; a nonionic surfactant; a thickening agent which is sodium carboxymethyl cellulose optionally admixed with a thickening silica; a humectant; and an abrasive which is a "low-anion" silica.

8. The use as claimed in any one of Claims 1 to 4 in which the composition is a mouthwash.

9. The use as claimed in any one of the preceding claims in which the composition further comprises an orally acceptable chelating agent.

10. The use as claimed in Claim 9 in which the orally acceptable chelating agent is EDTA, citric acid, or an alkali metal salt thereof.

## Patentansprüche

1. Die Verwendung von Nisin, einem Antibiotikum, das aus Chlorhexidin oder einem Salz davon, Cetylpyridinchlorid oder Triclosan ausgewählt wurde, und einem mundverträglichen Vehikel oder Träger bei der Herstellung einer Mundpflegezusammensetzung zur Behandlung oder Prophylaxe einer Mundfungalinfektion.

2. Die Verwendung gemäß Anspruch 1, dadurch gekennzeichnet, daß das Nisin in gereinigter Form verwendet wird.

3. Die Verwendung gemäß Anspruch 1 oder 2, dadurch gekennzeichnet, daß der mundverträgliche Träger oder Vehikel ein Detergens umfaßt, das ein nichtionisches, kationisches oder amphoteres Detergens oder ein Gemisch davon ist.

4. Die Verwendung gemäß jedem der Ansprüche 1 bis 3, dadurch gekennzeichnet, daß der mundverträgliche Träger oder Vehikel ein nichtionisches Verdickungsmittel oder einen natürlichen oder synthetischen Gummi oder ein gummiähnliches Material umfaßt.

5. Die Verwendung gemäß jedem der Ansprüche 1 bis 4, dadurch gekennzeichnet, daß die Zusammensetzung ein Zahnreinigungsmittel ist und daß das Schleifmittel ein Siliciumdioxidschleifmittel ist.

6. Die Verwendung gemäß jedem der Ansprüche 1 bis 4, dadurch gekennzeichnet, daß die Zusammensetzung ein Zahnreinigungsmittel ist, das Nisin, ein Antibiotikum, das aus Cetylpyridinchlorid, einem Chlorhexidinsalz oder Triclosan ausgewählt wurde, ein nichtionisches Detergens, ein nichtionisches Verdickungsmittel, ein Feuchthaltemittel und ein Schleifmittel umfaßt, das ein 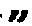 Niedrig-Anion"-siliciumdioxid oder Calciumcarbonat gegebenenfalls in Kombination mit Dicalcium-*ortho*monophosphat ist und ein Erdalkalimetallsalz wie Calciumchlorid einschließt.

7. Die Verwendung gemäß jedem der Ansprüche 1 bis 4, dadurch gekennzeichnet, daß die Zusammensetzung ein Zahnreinigungsmittel ist, das Nisin, ein Antibiotikum, das aus Cetylpyridinchlorid oder Triclosan ausgewählt wurde, ein nichtionisches Detergens, ein Verdickungsmittel, das Natriumcarboxymethylcellulose ist, der gegebenenfalls ein Verdickungssiliciumdioxid beigemischt ist, ein Feuchthaltemittel und ein Schleifmittel umfaßt, das ein 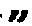 Niedrig-Anion"-siliciumdioxid ist.

8. Die Verwendung gemäß jedem der Ansprüche 1 bis 4, dadurch gekennzeichnet, daß die Zusammensetzung ein Mundwasser ist.

9. Die Verwendung gemäß jedem der vorstehenden Ansprüche, dadurch gekennzeichnet, daß die Zusammensetzung weiterhin einen mundverträglichen Chelatbildner umfaßt.

10. Die Verwendung gemäß Anspruch 9, dadurch gekennzeichnet, daß der mundverträgliche Chelatbildner EDTA, Zitronensäure oder ein Alkalimetallsalz davon ist.

## Revendications

1. Utilisation de nisine, agent antimicrobien choisi parmi la chlorhexidine ou un de ses sels, le chlorure de cétyl pyridinium ou le triclosan et d'un excipient ou véhicule acceptable par voie orale dans la préparation d'une composition de soin par voie orale destinée au traitement ou à la prophylaxie d'une infection fongique des voies orales.

2. Utilisation selon la revendication 1 caractérisée en ce que la nisine est utilisée sous une forme purifiée.

3. Utilisation selon la revendication 1 ou 2 caractérisée en ce que l'excipient ou le véhicule acceptable par voie orale comprend un tensioactif qui est un tensioactif non-ionique, cationique ou amphotère ou un mélange de ceux-ci.

4. Utilisation selon l'une quelconque des revendications 1 à 3 caractérisée en ce que l'excipient ou le véhicule acceptable par voie orale comprend un agent épaississant non-ionique ou une gomme naturelle ou synthétique ou une substance de type gomme.

5. Utilisation selon l'une quelconque des revendications 1 à 4 caractérisée en ce que la composition est un dentifrice et en ce que l'abrasif est un abrasif silicié.

6. Utilisation selon l'une quelconque des revendications 1 à 4 caractérisée en ce que la composition est un dentifrice qui comprend la nisine ; un agent antimicrobien choisi parmi le chlorure de cétyl pyridinium, un sel de chlorhexidine ou le triclosan ; un tensioactif non-ionique ; un agent épaississant non-ionique ; un humectant ; et un abrasif qui est une silice 〈〈 anionique faible 〉〉 ou le carbonate de calcium éventuellement associé à l'*ortho*monophosphate dicalcique et incluant un sel de métal alcalino-terreux tel que le chlorure de calcium.

7. Utilisation selon l'une quelconque des revendications 1 à 4 caractérisée en ce que la composition est un dentifrice qui comprend la nisine ; un agent antimicrobien choisi parmi le chlorure de cétyl pyridinium ou le triclosan ; un tensioactif non-ionique ; un agent épaississant qui est la carboxyméthylcellulose de sodium éventuellement en mélange avec une silice épaississante ; un humectant ; et un abrasif qui est une silice 〈〈 anionique faible 〉〉.

8. Utilisation, selon l'une quelconque des revendications 1 à 4 caractérisée en ce que la composition est un bain de bouche.

9. Utilisation, selon l'une quelconque des revendications précédentes caractérisée en ce que la composition comprend en outre un agent chélatant acceptable par voie orale.

10. Utilisation selon la revendication 9, caractérisée en ce que l'agent chélatant acceptable par voie orale est l'EDTA, l'acide citrique ou un de ses sels de métal alcalin.
